(19) Europäisches Patentamt
European Patent Office
Office européen des brevets

(11) Publication number: **0 054 309**
**B1**

(12) # EUROPEAN PATENT SPECIFICATION

(45) Date of publication of patent specification: **22.08.84**

(51) Int. Cl.$^3$: **C 07 D 217/20,** A 61 K 31/47

(21) Application number: **81110513.9**

(22) Date of filing: **16.12.81**

(54) **Long duration neuromuscular blocking agents, pharmaceutical compositions containing them and processes for their preparation.**

(30) Priority: **17.12.80 US 217444**

(43) Date of publication of application: **23.06.82 Bulletin 82/25**

(45) Publication of the grant of the patent: **22.08.84 Bulletin 84/34**

(84) Designated Contracting States: **BE DE FR GB IT NL**

(56) References cited:
**GB-A- 863 717**
**GB-A-1 579 822**
**US-A-4 192 877**

(73) Proprietor: **THE WELLCOME FOUNDATION LIMITED**
**183-193 Euston Road**
**London NW1 2BP (GB)**

(72) Inventor: **El-Sayad, Hassan Ali**
**214 Pinegate, Apt.10**
**Chapel Hill North Carolina 27514 (US)**
Inventor: **Yeowell, David Arthur**
**608 Concordia Court**
**Chapel Hill North Carolina 27514 (US)**
Inventor: **Swaringen, Roy Archibald, Jr.**
**824 Sandlewood Drive**
**Durham North Carolina 27712 (US)**

(74) Representative: **Berg, Wilhelm, Dr. et al**
**Dr. Berg, Dipl.-Ing. Stapf, Dipl.-Ing. Schwabe, Dr. Dr. Sandmair Mauerkircherstrasse 45**
**D-8000 München 80 (DE)**

**EP 0 054 309 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

In anesthesia, neuromuscular blocking agents are used to provide skeletal muscle relaxation during surgery and during intubation of the trachea.

In general there are two types of neuromuscular blocking agents in use, non-depolarizing and depolarizing.

The non-depolarizing agents include d-tubocurarine, pancuronium, gallamine, diallyltoxiferine and toxiferine.

The depolarizing agents includes succinylcholine and decamethonium. All of the conventional non-depolarizing agents when used for producing skeletal muscle relaxation in surgery have a long duration of action, e.g. 60 to 180 minutes in man. The conventional depolarizing agents, on the other hand, provide muscle relaxation with duration of action shorter than that of the non-depolarizing agents.

For example, succinylcholine provides a short duration of action of about 5 to 15 minutes whereas decamethonium provides about 20 to 40 minutes duration of muscle relaxation in man.

Each non-depolarizing agent has inherent side effects. For example gallamine and pancuronium may cause tachycardia, and d-tubocurarine and diallyltoxiferine may cause hypotension.

While these drugs can be pharmacologically antagonized with anticholinesterase agents, this obviously necessitates the administration of a second drug which itself may have its own side effects, e.g. bradycardia, gut spasm and bronchorrhea. Thus, to overcome the aforementioned side effects of the anticholinesterase agents, a third drug, an anticholinergic drug, e.g. atropine must also be given.

Surprisingly, the compounds of the present invention have a very high potency, long duration of action and are apparently free of any side effects at the dosages anticipated being used clinically. Furthermore, the *trans* compound where Y is methyl has shown unexpectedly superior activity (potency) and surprisingly much longer duration than any of their analogues, i.e., compounds with different chain lengths and the same isoquinoline base or compounds with different bases and the same chain length.

According to a first aspect of the present invention, there are provided new neuromuscular blocking agents (sometimes called muscle relaxtants) of the formula (I):

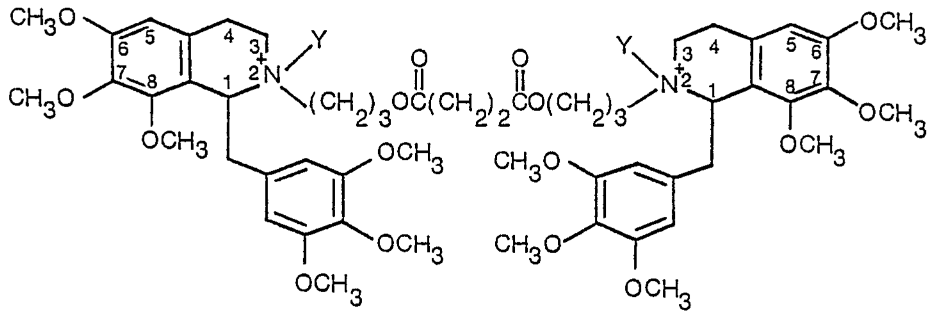

(I)

2X⁻

wherein Y represents a lower alkyl group of 1 to 4 carbon atoms (methyl, ethyl, propyl or butyl); $X^-$ is an anion, preferably pharmaceutically acceptable; and preferably the trimethoxybenzyl group at the 1 position and the proximal $(CH_2)_3$ (also known as substituted propyl) moiety of the group $(CH_2)_3OCO(CH_2)_2OCO(CH_2)_3$ at the 2 position are in a *trans* relationship with each other in each nitrogen-containing ring.

The preferred compound is that wherein Y represents a methyl group.

Since the activity of the compounds of the invention resides in the di-cation, the nature of the anion $X^-$ is relatively unimportant. Suitable pharmaceutically acceptable anions include iodide, mesylate, tosylate, bromide, chloride, sulphate, phosphate, hydrogen phosphate, acetate, benzenesulphonate, succinate, maleate, naphthalenesulphonate and propionate.

The compounds of the invention are preferably prepared as a mixture of the racemic (dl) pair and the *meso*-isomer suitably in a ratio of 1:1. This invention further provides means for obtaining the compounds of formula (I) when in the form of one of the aforesaid isomers substantially free of the other isomers, and mixtures of one of the isomers with one or both of the other isomers. Other methods of preparing the *cis-trans* mixtures are well known in the art.

It is preferred that the compounds of the invention be provided in a form where the ratio of the *trans, trans* compound of the invention to the total of any corresponding *cis, cis* and *cis, trans* compounds present as impurities is at least 96:4.

The compounds of formula (I) may be used as neuromuscular blocking agents in conjunction with surgery or for intubation of the trachea by conventional parenteral administration, e.g. intramuscular or intravenous administration in solution. According to a second aspect of the present invention, therefore, there is provided a compound in accordance with the first aspect for use as a neuromuscular blocking

agent. The compounds of the present invention shown in formula (I) are administered to subjects such as monkeys and man (humans) and other mammals to achieve a neuromuscular block. The dosage for each type of subject will vary because of the peculiarities of the species. However, a suitable intravenous amount of dosage of the compounds of formula (I) to obtain paralysis in mammals would be 0.004 to 0.03 mg/kg of body weight, and most preferably 0.01 to 0.02 mg/kg of body weight, the above being based on the weight of the di-cation which is the active ingredient. The compounds of this invention appear, therefore, to be clearly more potent than the agents most widely used clinically (pancuronium 0.06—0.08 mg/kg, d-tubocurarine 0.4—0.6 mg/kg). The dosage for intramuscular administration is two to four times the intravenous dose. The compounds of this invention are reversible using conventional anti-cholinesterase agents such as neostigmine and edrophinium and appear to avoid the side effects associated with the non-depolarizing agents.

The compounds of formula (I) are therefore useful for producing a long duration neuromuscular blockade in man as well as in other mammals, and the present invention provides a method of producing such blockade in mammals by intravenously injecting a dose of 0.004 to 0.03 mg/kg to the mammal. It should be understood that the duration in a mammal such as monkey is considerably shorter than in humans and is considered a long duration agent for that species.

The compounds may be presented in a pharmaceutical formulation for parenteral administration. The formulations may be an aqueous or non-aqueous solution or emulsion in a pharmaceutically acceptable liquid or mixture of liquids, which may contain bacteriostatic agents, antioxidants, buffers, thickening agents, suspending agents or other pharmaceutically acceptable additives. Such formulations are normally presented in unit dosage forms such as ampoules or disposable injection devices, or in multidose forms such as a bottle from which the appropriate dose may be withdrawn; all such formulations should be sterile.

The compounds of this invention may be presented as a powder, e.g. as a unit dose in a sealed vial to which sterile water or an other pharmaceutically acceptable sterile liquid vehicle may be added, preferably by aseptic techniques.

A suitable unit dose to obtain a neuromuscular block for adult humans (~150 lb) is about 0.15 mg to 2.5 mg and most preferably 0.5 to 1.5 mg.

The compounds of this invention if desired may be administered in conjunction with depolarizing agents such as listed above.

Thus a suitable pharmaceutical parenteral preparation for administration to humans will preferably contain 0.3 to 2.5 mg of the compounds of formula (I) of this invention in solution.

A simple and preferred formulation is a solution of the compound of formula (I) in water which may be prepared by simply dissolving the compound into previously sterilized pure water (i.e. pyrogen free water), under aseptic conditions, and sterilizing the solution.

The compound of formula (I) may also be administered as an infusion of a dextrose solution or a saline solution, e.g. Ringer's solution.

The compounds may also be administered in other solvents such as alcohol, polyethylene glycol and dimethylsulphoxide. They may also be administered intramuscularly as a suspension.

According to another aspect of the present invention, there is provided a process for the preparation of the compounds of formula (I), as defined above, which process comprises either

A. effecting the coupling of two *trans*-N-($C_1$ to $C_4$)alkyl-N-3-hydroxypropyl-1,2,3,4-tetrahydro-6,7,8-trimethoxy-1-(3,4,5-trimethoxybenzyl)-isoquinolinium ions with succinic acid or a reactive derivative thereof; or

B. effecting the coupling of two *trans*-N-($C_1$ to $C_4$)alkyl-N-3-halopropyl-1,2,3,4-tetrahydro-6,7,8-trimethoxy-1-(3,4,5-trimethoxybenzyl)-isoquinolinium ions with the di-silver salt of succinic acid.

Suitable reactive derivatives of succinic acid are succinic anhydride and succinyl chloride.

The coupling of the 3-halopropyl compounds using the disilver slat of succinic acid may be carried out in a manner similar to that described in US Patent No. 4,192,877.

For a better understanding of the present invention, the following Examples are given.

Example 1A

5′,8-Dimethoxylaudanosine (27.2 g) and iodopropanol (27.2 g) were refluxed in 150 ml of dry acetone for 21 hours. The solvent was evaporated under vacuum and the unreacted iodopropanol was extracted with 100 ml of diethyl ether. The ether was decanted and the residue was dissolved in 300 ml of hot ethyl alcohol and cooled at 5° for 16 hours to yield 29.2 g of a 9/1 mixture of the *trans-cis* quaternary iodides as indicated by High Performance Liquid Chromatography (HPLC). The mixture was recrystallized twice from ethyl alcohol to give 24.4 g of *trans*-N-3-hydroxypropyl-5′,8-dimethoxy-laudanosinium iodide (98% *trans* by HPLC). The iodide salt was converted to the chloride salt by passing its methanolic solution through a column packed with 75 g of Dowex 1—X8 ion exchange resin. The solvent was evaporated under vacuum and 100 ml of acetone was added to give 18.1 g of *trans*-N-3-hydroxypropyl-5′,8-dimethoxylaudanosinium chloride (100% *trans* by HPLC). The yield was 67% overall.

Calculated for $C_{26}H_{38}NO_7Cl_22H_2O$: C, 56.98; H, 7.72; N, 2.56; Cl, 6.47. Found: C, 56.97; H, 7.74; N, 2.52; Cl, 6.47.

'Dowex' is a Trade Mark.

Example 1B

*Trans*-N-3-hydroxypropyl-5',8-dimethoxylaudanosinium chloride (99% *trans* by HPLC, 2 g) was suspended in 150 ml of 1,2-dichloroethane at ~70° and succinyl chloride (0.24 g) was added. The mixture was heated at reflux for 140 minutes. The solvent was removed under vacuum to give an amorphous solid which was dissolved in 100 ml of chloroform and washed with 5% aqueous sodium chloride solution 8 × 100 ml to remove the unreacted quaternary salt. The chloroform layer was washed with 50 ml of water, dried and evaporated under vacuum. The residual amorphous solid was dissolved in water and lyophilized to give 0.51 g of *trans, trans*-2,2'-(dimethylene*bis*(carbonyloxytrimethylene))*bis* (1,2,3,4-tetrahydro-6,7,8-trimethoxy-2-methyl-1-(3,4,5-trimethoxybenzyl)isoquinolinium) dichloride which was assayed by High Performance Liquid Chromatography (HPLC) as 100%.

Calculated for $C_{56}H_{78}N_2O_{16}2Cl\ 6\ H_2O$: C, 55.39; H, 7.47; N, 2.31; Cl, 5.83. Found: C, 55.72; H,7.04; N, 2.27; Cl, 5.84.

Example 1C

Mescaline and 3,4,5-trimethoxyphenylacetic acid were reacted in xylene to give the corresponding amide which was cyclized to the corresponding dihydroisoquinoline via the Bischler-Napieralski reaction followed by reduction and reductive methylation to give 5',8-dimethoxylaudanosine mp 174—176.

Example 2

*trans, trans*-2,2'-(Dimethylene*bis*(carbonyloxytrimethylene))-*bis*(1,2,3,4-tetrahydro-6,7,8-trimethoxy-2-methyl-1-(3,4,5-trimethoxybenzyl)isoquinolinium) dichloride was examined by intravenous administration to cats and Rhesus monkies, maintained by artifical ventilation and prepared for recording the isometric twitch of the tibialis anterior muscle in responce to stimulation of the peroneal nerve. The results are shown in the following tables.

| Cat | | | Rhesus Monkey | | |
|---|---|---|---|---|---|
| No. of Animals | $ED_{95}$ mg/kg | Duration Minute | No. of Animals | $ED_{95}$ mg/kg | *Duration Minute |
| 2 | 0.01 | ~60 | 2 | 0.015 | ~30⁺ |

* The time from injection to 95% recovery
⁺ This translates to about 90 minutes in man.

**Claims**

1. A compound characterized by formula (I):

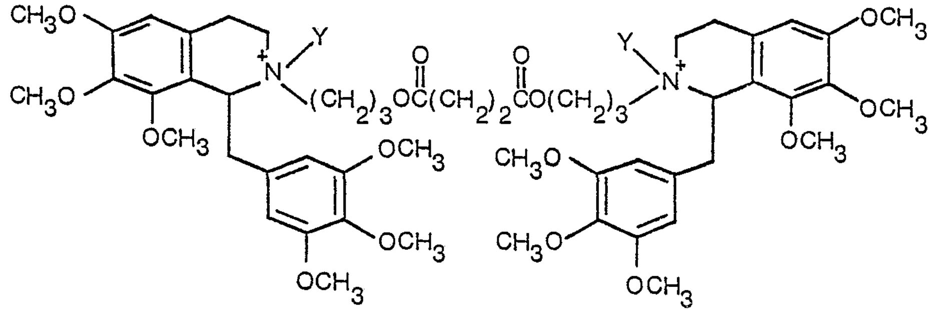

(I)

2X⁻

wherein Y represents lower alkyl group of 1 to 4 carbon atoms and X represents an anion.

2. A compound according to Claim 1, characterized in that, for each of the nitrogen containing rings, the trimethoxybenzyl group at the 1 position and the group $(CH_2)_3OCO(CH_2)_2OCO(CH_2)_3$ at the 2 position are in a *trans* relationship with each other.

3. A compound according to Claim 1 or 2, characterized in that Y represents a methyl group.

4. A compound according to Claim 1 or 2, characterized in that X represents a pharmaceutically acceptable anion.

5. A mixture comprising the mixture of the racemic (dl) pair and the meso-isomer of the compound characterized by formula (I):

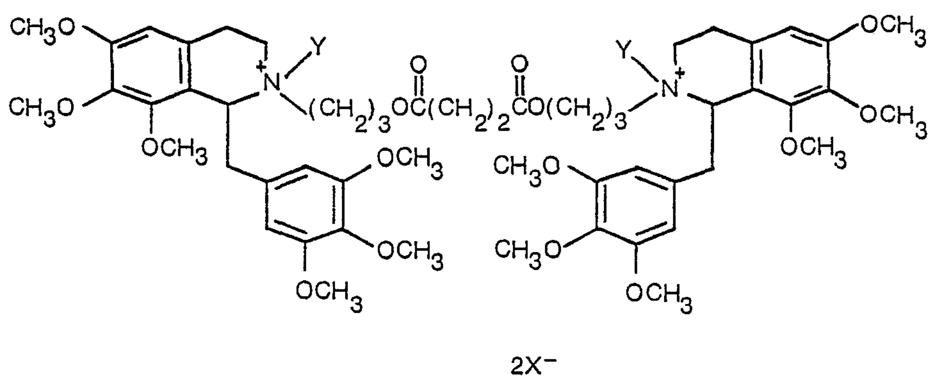

(I)

wherein Y represents a lower alkyl group of 1 to 4 carbon atoms and X represents an anion.

6. A mixture according to Claim 5, characterized in that Y represents a methyl group and X represents a pharmaceutically acceptable anion.

7. A compound characterized by formula (I)

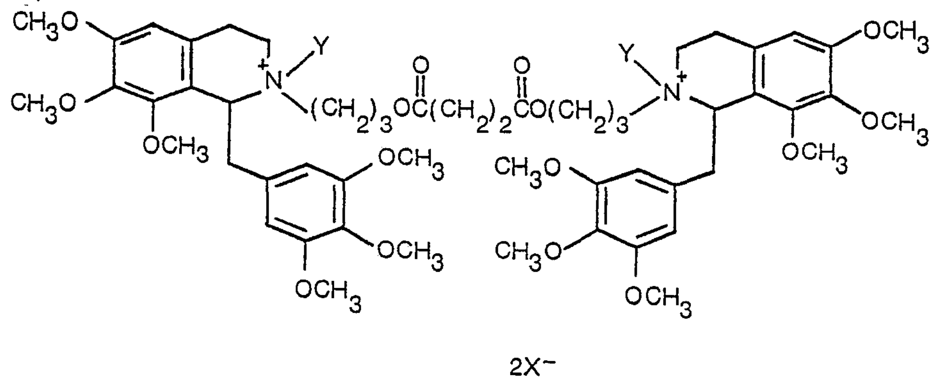

(I)

wherein Y represents a lower alkyl group of 1 to 4 carbon atoms and X represents an anion, for use as a neuromuscular blocking agent.

8. A pharmaceutical composition comprising one or more active ingredient and a pharmaceutically acceptable carrier, characterized in that the or each or an active ingredient is a compound of formula (I).

9. A process for preparing a compound of formula (I):

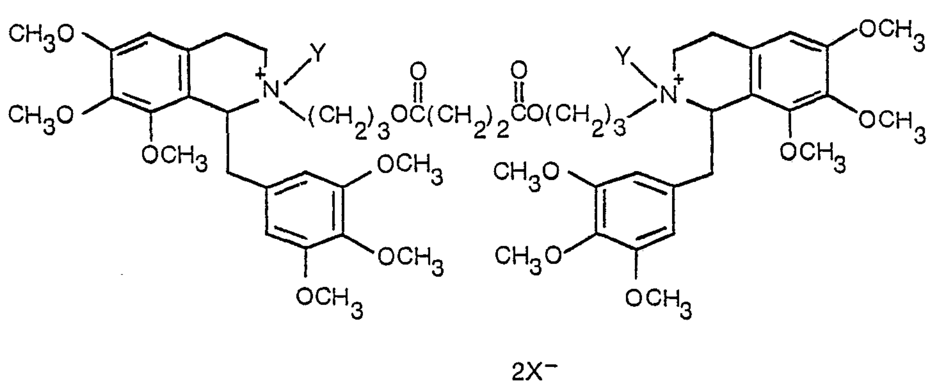

(I)

characterized by either

A. effecting the coupling of two *trans*-N-($C_1$ to $C_4$)alkyl-N-3-hydroxypropyl-1,2,3,4-tetrahydro-6,7,8-trimethoxy-1-(3,4,5-trimethoxybenzyl)-isoquinolinium ions with succinic acid or a reactive derivative thereof; or

B. effecting the coupling of two *trans*-N-($C_1$ to $C_4$)alkyl-N-3-halopropyl-1,2,3,4-tetrahydro-6,7,8-trimethoxy-1-(3,4,5-trimethoxybenzyl)-isoquinolinium ions with the di-silver salt of succinic acid.

10. A process according to Claim 9A, characterized in that the coupling is effected with succinic anhydride or succinyl chloride.

**Patentansprüche**

1. Verbindung, gekennzeichnet durch Formel I

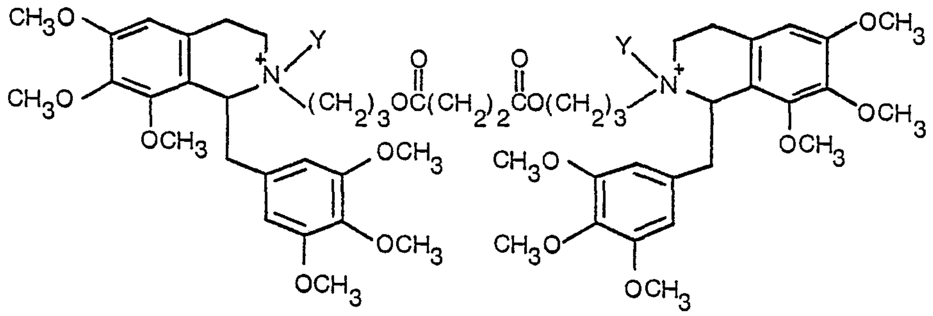

(I)

$2X^-$

worin Y eine Niederalkylgruppe mit 1 bis Kohlenstoffatomen bedeutet und X ein Anion darstellt.

2. Verbindung nach Anspruch 1, dadurch gekennzeichnet, daß für jeden der Stickstoff enthaltenden Ringe die Trimethoxybenzylgruppe in der 1-Stellung und die Gruppe $(CH_2)_3OCO(CH_2)_2OCO(CH_2)_3$ in der 2-Stellung in einer trans-Beziehung zueinander stehen.

3. Verbindung nach Anspruch 1 oder 2, dadurch gekennzeichnet, daß Y eine Methylgruppe darstellt.

4. Verbindung nach Anspruch 1 oder 2, dadurch gekennzeichnet, daß X ein pharmazeutisch verträgliches Anion bedeutet.

5. Gemisch, enthaltend das Gemisch des racemischen (dl)-Paars und des Meso-Isomers der Verbindung, die durch die Formel I

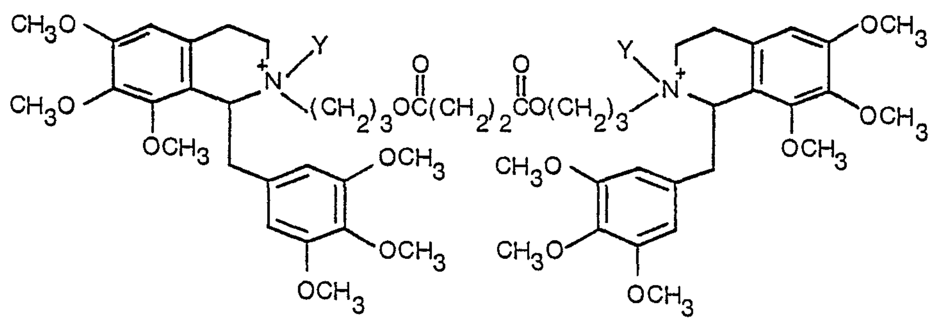

(I)

$2X^-$

gekennzeichnet ist, worin Y eine Niederalkylgruppe mit 1 bis 4 Kohlenstoffatomen bedeutet und X ein Anion darstellt.

6. Gemisch nach Anspruch 5, dadurch gekennzeichnet, daß Y eine Methylgruppe darstellt und X ein pharmazeutisch verträgliches Anion bedeutet.

7. Verbindung, gekennzeichnet durch die Formel I,

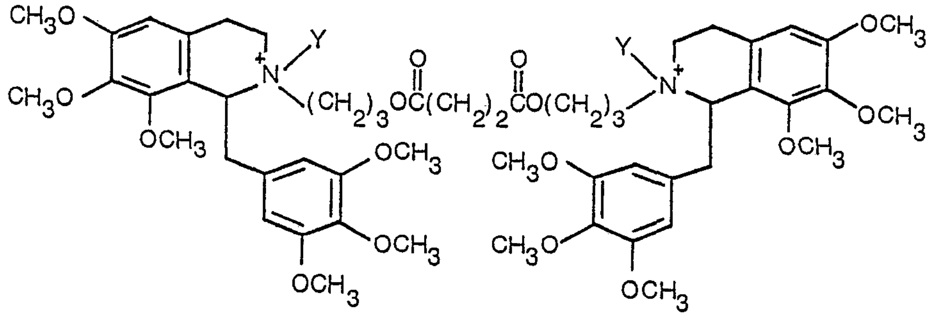

(I)

$2X^-$

worin Y eine Niederalkylgruppe mit 1 bis 4 Kohlenstoffatomen darstellt und X ein Anion bedeutet, zur Verwendungs als ein neuromuskuläres Blockierungsmittel.

Kohlenstoffatomen darstellt und X ein Anion bedeutet, zur Verwendungs als ein neuromuskuläres Blockierungsmittel.

8. Pharmazeutische Zusammensetzung, enthaltend einen oder mehrere aktive Bestandteile und einen pharmazeutisch unbedenklichen Träger, dadurch gekennzeichnet, daß der oder jeder der aktiven Bestandteile eine Verbindung der Formel I ist.

9. Verfahren zur Herstellung einer Verbindung der Formel I

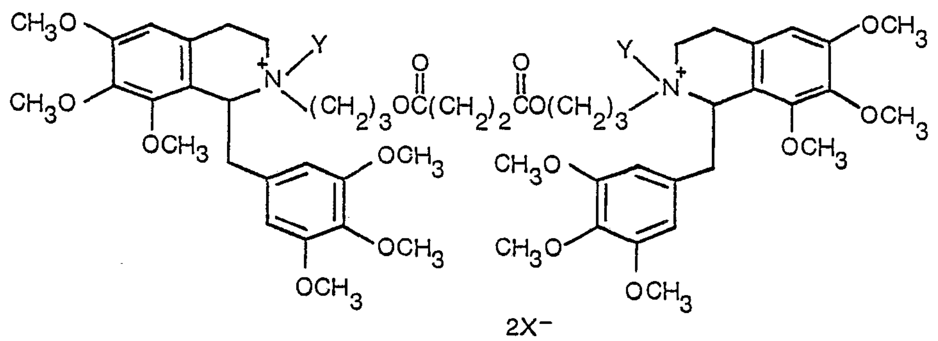

(I)

$2X^-$

dadurch gekennzeichnet, daß entweder

A. die Kupplung zweier trans-N-($C_1$ bis $C_4$)-Alkyl-N-3-hydroxypropyl-1,2,3,4-tetrahydro-6,7,8-trimethoxy-1-(3,4,5-trimethoxybenzyl)-isochinolinionen mit Succinsäure oder einem rekativen Derivat davon durchgeführt wird oder

B. die Kupplung von zwei trans-N-($C_1$ bis $C_4$)-Alkyl-N-3-halogenpropyl-1,2,3,4-tetrahydro-6,7,8-trimethoxy-1-(3,4,5-trimethoxybenzyl)-isochinolinionen mit dem Disilbersalz der Succinsäure durchgeführt wird.

10. Verfahren nach Anspruch 9A, dadurch gekennzeichnet, daß die Kupplung mit Succinsäureanhydrid oder Succinylchlorid bewirkt wird.

**Revendications**

1. Composé, caractérisé par la formule (1):

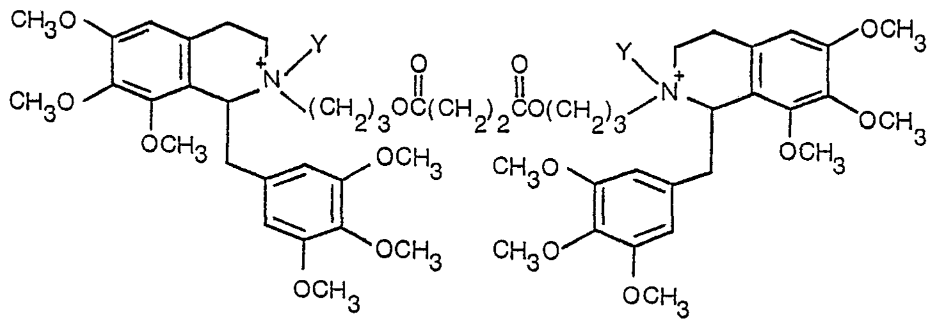

(I)

$2X^-$

où Y représente un radical alcoyle inférieur de 1 à 4 atomes de carbone et $X^-$ représente un anion.

2. Composé suivant la revendication 1, caractérisé en ce que pour chaque cycle contenant de l'azote le radical triméthoxybenzyle à la position 1 et le radical $(CH_2)_3OCO(CH_2)_2OCO(CH_2)_3$ à la position 2 sont en relation *trans* l'un par rapport à l'autre.

3. Composé suivant la revendication 1 ou 2, caractérisé en ce que Y représente un radical méthyle.

4. Composé suivant la revendication 1 ou 2, caractérisé en ce que X représente un anion pharmaceutiquement acceptable.

5. Mélange, qui comprend le mélange de la paire racémique (dl) et du mésoisomère de composé caractérisé par la formule (1):

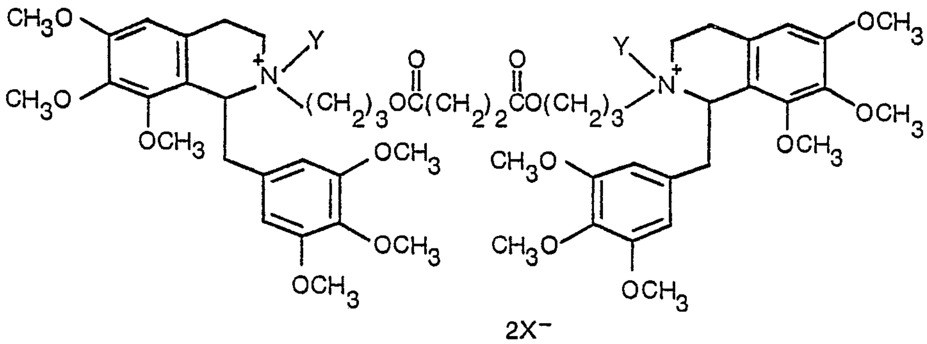

(I)

où Y représenté un radical alcoyle inférieur de 1 à 4 atomes de carbone et X représente un anion.

6. Mélange suivant la revendication 5, caractérisé en ce que Y représente un radical méthyle et X représente un anion pharmaceutiquement acceptable.

7. Composé, caractérisé par la formule (1):

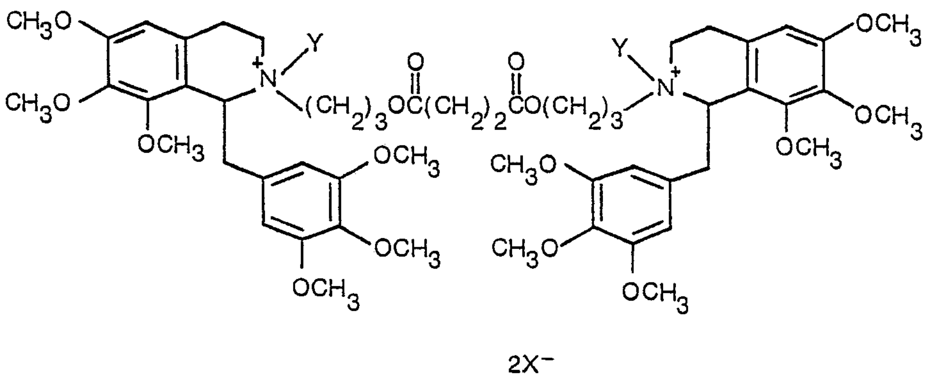

(I)

où Y représente un radical alcoyle inférieur de 1 à 4 atomes de carbone et X représente un anion, à utiliser comme bloquant neuromusculaire.

8. Composition pharmaceutique comprenant un ou plusieurs principes actifs et un excipient pharmaceutiquement acceptable, caractérisée en ce que le ou chaque principe actif est un composé de formule (1).

9. Procédé de préparation d'un composé de formule (1):

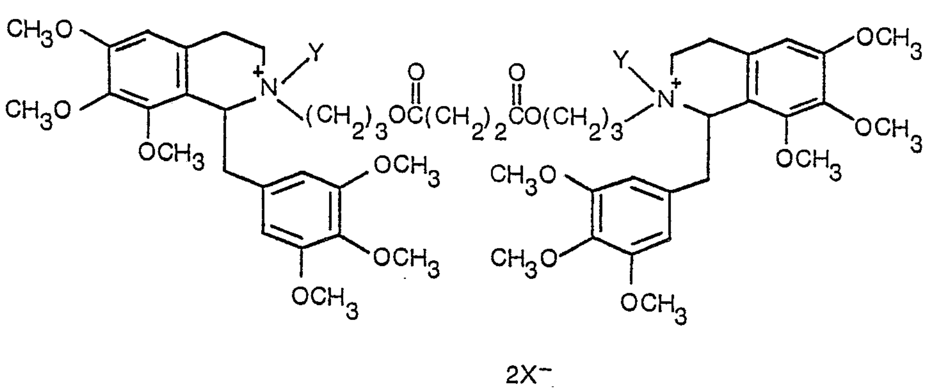

(I)

caractérisé en ce que

A. on exécute la condensation de deux ions *trans*-N-($C_1$ à $C_4$)alcoyl-N-3-hydroxypropyl-1,2,3,4-tétrahydro-6,7,8-triméthoxy-1-(3,4,5-triméthoxybenzyl)-isoquinoléinium avec l'acide succinique ou un dérivé réactif de celui-ci; ou bien

B. on exécute la condensation de deux ions *trans*-N-($C_1$ à $C_4$)alcoyl-N-3-halopropyl-1,2,3,4-tétrahydro-6,7,8-triméthoxy-1-(3,4,5-triméthoxybenzyl)-isoquinoléinium avec le sel diargentique d'acide succinique.

10. Procédé suivant la revendication 9, caractérisé en ce qu'on exécute la condensation avec l'anhydride succinique ou le chlorure de succinyle.